# EUROPÄISCHE PATENTSCHRIFT

(11) **EP 2 161 216 B1**
(45) Veröffentlichungstag und Bekanntmachung des Hinweises auf die Patenterteilung: **23.05.2012**
(21) Anmeldenummer: 09013914.8
(22) Anmeldetag: 13.03.2009
(51) Int. Cl.: B65D 83/00, G01F 23/26, B05C 17/015, A61M 5/162, B65D 75/32, B67D 7/02

(54) **Behälter für fließfähige Substanzen und Verfahren zum blasenfreien Dosieren solcher Substanzen**
Container for flowable substances and method for non-porous metering of such substances
Récipient pour substances pouvant s'écouler et procédé de dosage sans bulle de telles substances

(30) Priorität: 18.03.2008 DE 102008014773
(43) Veröffentlichungstag der Anmeldung: 10.03.2010
(62) Teilanmeldung aus: 09723450.4
(73) Patentinhaber: Delo Industrieklebstoffe Gmbh & Co. Kg Aa, 86949 Windach (DE)
(72) Erfinder: Herold, Wolf, 86911 Diessen (DE); Neuhaus, Stephan, 86153 Augsburg (DE)
(74) Vertreter: Strehl Schübel-Hopf & Partner

(56) Entgegenhaltungen:
- DE-A1- 19 513 223
- US-A- 4 282 986

## Beschreibung

### Stand der Technik

Bei der Dosierung von fließfähigen Substanzen (z.B. Klebstoffen, Dicht- und Formmassen) in Anwendungsbereichen der Elektronik, der Dentalmedizin oder der Verbindungstechnik allgemein, ist das Aufbringen einer absolut blasenfreien Substanz von entscheidender Bedeutung. Insbesondere bei einer automatisierten Fertigung, bei der kleinste Dosiermengen der Substanz auf ein Substrat aufgebracht werden, erzeugen Luftblasen im Klebstoff fehlerhafte Bauteile und damit Ausschuss.

Nicht nur, dass anschließend eine Kontrolle aller Bauteile durchzuführen ist, um fehlerbehaftete Teile auszusortieren, sondern zusätzlich muss in der Regel beim Auftreten von Luftblasen die gesamte Anlage angehalten und zeitaufwändig gereinigt werden. Je kleiner die Dosiervolumina sind, desto größer wird der Ausschuss beim Auftreten einer Luftblase.

Hochwertige Kleb- und Dichtstoffe sowie dentale Abformmaterialien werden dem Anwender häufig in einer handelsüblichen, starren Einwegkartusche als Primärbehältnis angeboten. Das Auspressen dieser Kartusche erfolgt durch Vorschieben eines Kolbens, entweder mechanisch oder über Druckluft. Die Erfahrung zeigt, dass blasenfrei befüllte und blasenfrei verschlossene Kartuschen beim Kunden voller Blasen eintreffen.

Die Erklärung ist wie folgt: Viele einkomponentige Klebstoffe müssen zur Vermeidung einer frühzeitigen Aushärtung, während der gesamten Lagerzeit gekühlt oder tiefgefroren werden. Bei gegenüber der Raumtemperatur abgesenkten Temperaturen schrumpft auf Grund unterschiedlicher thermischer Ausdehnungskoeffizienten die abgefüllte Flüssigkeit stärker als der starre Kunststoff des Primärbehältnisses. Bei tiefen Temperaturen wird aus der flüssigen Substanz ein fester Block, der sich teilweise von der Wandung der Kartusche löst. Bei diesem Vorgang wird in der Kartuschenkammer ein Unterdruck erzeugt, der dann über den reibungsbehafteten, festsitzenden Verschlusskolben oder eventuell auch den vorderen Verschluss Luft in die Kartusche saugt. Beim Wiederauftauen des Kartuscheninhalts wird man in der gesamten flüssigen Substanz Luftblasen detektieren können. Bei mehrmaligem Einfrieren und Auftauen der Kartusche wird, bedingt durch das allmähliche Zurückgleiten des Verschlusskolbens, bei jedem Vorgang von mal zu mal mehr Luft in die Füllkammer eingesogen.

Den gleichen Vorgang beobachtet man bei einer Substanz, die nicht tiefgefroren werden muss. Lagert man einen bei Raumtemperatur abgefüllten Klebstoff bei höherer Temperatur (z.B. im Sommer), so wird er sich ausdehnen. Dabei schiebt er den reibungsbehafteten aber beweglichen Verschlussstopfen nach hinten. Bei anschließender Abkühlung auf Raumtemperatur schrumpft der Klebstoff. Auf Grund seiner Reibung verharrt jedoch der Verschlusskolben in seiner zurückgeschobenen Stellung. Es entsteht somit ein Unterdruck, der Luft am Kolben vorbei in die Füllkammer saugt. Wird diese Kartusche per Luftfracht versandt, wandert der Verschlusskolben bei jedem Start- und Landevorgang bedingt durch den Temperaturwechsel deutlich nach hinten.

Letztlich muss man davon ausgehen, dass ursprünglich absolut blasenfrei abgefüllte Kartuschen nach Versand und Lagerung dem Anwender nicht blasenfrei zur Verfügung stehen und der Einsatz dieser Kartuschen in der Fertigung zu gravierenden Problemen führt.

Eine ursprünglich blasenfreie, viskose Flüssigkeit kann auch voller Blasen sein, sofern man ein offenes Gebinde (z.B. eine offene Flasche oder eine Kartusche ohne Kolben) für den Dosiervorgang über einen längeren Zeitraum einer Druckluft aussetzt. Mit der Zeit werden sich immer mehr Anteile des Druckgases in der Substanz einlösen. Sobald diese Substanz nach dem Austreten aus dem Dosierventil dem normalen Atmosphärendruck ausgesetzt ist, dehnt sich das eingelöste Gas aus, und man findet viele kleine Luftblasen in der ehemals blasenfreien Substanz.

Die gleiche Erscheinung beobachtet man auch bei einer befüllten Kartusche, die einen Kolben aufweist und mittels Druckluft entleert wird. Sofern der Kolben bei seiner Vorwärtsbewegung gegenüber der Kartuschenwand eine Undichtigkeit aufweist, wird die Druckluft am Kolben vorbei zur Flüssigkeit gelangen und sich über die Zeit in das Material einlösen.

Lösungsansätze für eine permanent gesicherte Blasenfreiheit einer fließfähigen Substanz in einem Dosierbehältnis auch bei wechselnden Temperaturen lassen sich finden, sofern man die starre Wandung einer Kartusche durch eine flexible Folie ersetzt. So befindet sich die fließfähige Substanz gemäß DE 103 11 080 A1 zwar immer noch in einer herkömmlichen starren Kartusche. Der Verschlusskolben besteht aber aus einem festen äußeren Ring und der Kolbenboden aus einer flexiblen Folie. Thermisch bedingte Volumenveränderungen der Substanz nimmt die flexible Folie auf, ohne dass der Kolben verschoben wird. Nachteil dieser Lösung ist, dass bei Entleerung mittels Druckluft, Luft zwischen Kolbenring und Kartuschenwand in das Füllmedium gelangen kann. Umgekehrt kann beim mechanischen Auspressen das Füllmedium bei hohen Drücken am Kolben vorbei nach hinten ausgedrückt werden, was Verschmutzung und Substanzverlust bedeutet.

Marktübliche Verpackungen für fließfähige Substanzen, z.B. Dentalmassen und Kleb- und Dichtstoffen, sind dünne Verbundfolien, die vorn und hinten durch einen Metallclip verschlossen werden, vgl. EP 0 541 972 A1, DE 91 03 038 U1, EP 0 787 655 A1, DE 43 35 970 A1. Der Abfüllvorgang erfolgt blasenfrei auf einer herkömmlichen "Wurstbefüllanlage".

Um eine exakte und saubere Entleerung zu gewährleisten, werden diese zylindrischen Schlauchbeutel an einem Ende mit einem Ausbringstutzen versehen, der in der Regel aufgeschoben und verklebt wird. Zur Entleerung wird die Folie im Bereich des Austrittsstutzens mechanisch aufgeschnitten.

Dieser Typ eines Folienbehälters hat zwei wesentliche Nachteile:
- Es hat sich gezeigt, dass die Metallverschlüsse an den Enden gegenüber dünn fließenden Materialien oder Komponenten niemals dicht sind. Dies ist darauf zurückzuführen, dass man die zu einem Schlauch geformte Verbundfolie von einem großen Durchmesser auf einen sehr kleinen Durchmesser reduzieren muss. Die dabei zwangsläufig entstehenden Falten lassen auch bei sehr kräftigen Verschlussclips geringe Mengen an Flüssigkeit nach außen entweichen. Bei längerer Lagerzeit kapillieren niedrigviskose Bestandteile des Füllmaterials nach außen und verschmutzen den gesamten Beutel. Um die Verkaufsverpackung und die Hände des Anwenders beim Auspacken zu schützen, werden Folienbehälter dieser Art vornehmlich in Kunststoffbeuteln versandt. Nachteilig ist weiterhin dass sich durch das Entweichen der niedrigviskosen Komponente, die Zusammensetzung des ursprünglich abgefüllten Materials verändert.
   Das Austreten einer flüssigen Komponente an den undichten Stellen dieses Folienbehälters verstärkt sich beim Auspressen des Behälters in einer Ausbringvorrichtung. Dann wirken hohe Auspresskräfte auf den Folienbehälter und die unter diesem Druck austretende Flüssigkeit verschmutzt die Ausbringvorrichtung.
- Der zweite wesentliche Nachteil dieses Folienbehälters im Hinblick auf eine blasenfreie Dosierung ist darin zu sehen, dass zwischen der am Behälterende gefalteten Folie und dem angeklebten Ausbringstutzen in den Folienfalten Luft eingeschlossen ist, die sich nicht entfernen lässt. Beim automatischen Aufstechen eines Schlauchbeutels mittels eines Doms (EP 0 787 655 A1) befindet sich zusätzlich ein großes Luftvolumen zwischen dem Folienbeutel und dem Ende des Austrittsnippels. Beim Entleeren des Folienbehälters wird diese Luft, getrieben von den Auspresskräften, zu nicht vorhersehbaren Zeiten in Form von Luftblasen entweichen und Ausschuss produzieren.

Weitere ähnliche Folienbehälter zeigen JP 07 171 461 A und EP 1 331 174 A1.

Die marktüblichen Folienbehälter haben als Gemeinsamkeit einen zylindrischen Folienschlauch, der in der Regel zu einem Schlauch gefaltet und zusammengeschweißt ist. Der Auspressvorgang erfolgt entweder mit Druckluft oder durch einen mechanisch angetriebenen Kolben. Um zu verhindern, dass bei hohen Auspresskräften die dünne Folie des Behälters oder aber die Schweißnaht aufreißt, wird der Folienbehälter in eine stabile, zylindrische Hülse gesteckt. Der Innendurchmesser der Hülse und der Außendurchmesser des Folienbehälters sind sehr genau aufeinander abzustimmen. So verhindert ein Übermaß am Folienschlauch das Einschieben in die Hülse, während ein zu starkes Untermaß die Folie beim Auspressen reißen lässt.

Beim Ausdrücken der Kartusche wird die Schlauchfolie des Behälters durch den entstehenden Innendruck mit großer Kraft an die Wandung der Hülse gepresst. Gleichzeitig erfolgt mit dem Entleervorgang der Kartusche ein axiales Verschieben der Folie längs der Hülsenwand, wobei sich die Folie unkontrolliert zusammenfaltet. Dies bedeutet, dass beim Auspressen hohe Reibungskräfte entstehen, die der Auspresskraft entgegenwirken.

Diese Reibungskräfte sind zum einen abhängig von der Viskosität des Füllmaterials, der Auspresskraft, dem Überlappungsbereich der zweilagig verschweißten Folien und dem Untermaß des Außendurchmessers des Folienschlauchs. Weiterhin sind sie sehr abhängig von dem eigentlichen Entleervorgang. So sind diese Kräfte am Anfang des Zusammenschiebens des Folienschlauchs gering, nehmen während des Entleervorgangs zu und steigen zum Ende des Entleervorgangs extrem an.

Sofern das Auspressen der Kartusche mittels konstanter Druckluft erfolgt, werden die über eine definierte Zeiteinheit ausgebrachten Dosiermengen auf Grund der erwähnten Reibungseffekte sehr unterschiedlich sein. Damit scheidet eine solche Vorrichtung für die meisten Dosierungen prinzipiell aus. Selbst bei mechanischem Vorschub muss mit starken Schwankungen der Ausbringmengen gerechnet werden.

Nachteile ergeben sich auch für die Restentleerung. Durch die ungleichmäßige Faltenbildung der Folie während des Entleervorgangs, bilden sich abgeschlossene Kammern, in denen sich Füllmaterial befindet, das dann nicht mehr ausgepresst werden kann.

Ein Behälter, wo eine flexible Alu-Folie mittels Klebstoff an der innenwand einer stauen kugel fest fixiert wird, wird in der US-A-3 945 539 offenbart.

Ein Behälter für viskoses Füllgut ist aus DE 195 13 223 A1 bekannt. Das Füllgut befindet sich dort in einem aus flexible Kunststofffolien gebildeten Außenbehälter, in dem ein ebenfalls flexibler, aufblasbarer zur Entleerung Innenbehälter angeordnet ist.

Aus CH 605 328 A5 ist ein auch von älteren Patienten einfach zu handhabender Arzneimittelbehälter bekannt, der aus einem mit einem Auslass versehenen steifen konkaven Unterteil und einem mit diesem dicht verbundenen Oberteil aus einer zur Innenkontur des Unterteils im Wesentlichen spiegelbildlich konvex geformten flexiblen Folie besteht.

### Abriss der Erfindung

Der Erfindung liegt die Aufgabe zugrunde, einen Dosierbehälter für fließfähige Substanzen und anzugeben, bei dem Blasenfreiheit der Substanz während Lagerung und Transport auch bei stark wechselnden Temperaturen und Drücken sowie beim Entleervorgang und bei einem Behälterwechsel sichergestellt ist.

Weiterhin soll sichergestellt sein, dass bei gleichmäßigem Auspressdruck bei einer Zeitsteuerung über eine bestimmte Zeiteinheit gleichmäßige Dosiermengen ausgebracht werden. Diese Gleichmäßigkeit der ausgebrachten Dosiermenge soll über den gesamten Dosierprozess vom vollen Behälter bis zum fast leeren Behälter erhalten bleiben.

Zur erfindungsgemäße Lösung dieser Aufgabe dient der in Anspruch 1 angegebene Behälter.

Vorteilhafte Ausgestaltungen der Erfindung ergeben sich aus den Unteransprüchen.

### Kurzbeschreibung der Zeichnungen

In den Zeichnungen zeigt
Figur 1 einen Längsschnitt durch den Hauptteil eines gefüllten Dosierbehälters zur Erläuterung des der Erfindung zugrunde liegenden Prinzips,
Figur 2 einen der Figur 1 ähnlichen Schnitt durch den Behälter vor dem Befüllen,
Figur 3 einen der Figur 1 ähnlichen Schnitt durch den Behälter samt Druckgehäuse,
Figur 4 einen Teilschnitt durch das Behälterunterteil im ungefüllten Zustand,
Figur 5 einen der Figur 4 ähnlichen Teilschnitt durch das Behälterunterteil mit unterbrochenen rinnenförmigen Vertiefungen,
Figur 6 einen Schnitt durch die in der Nähe des Auslasses an mehreren Stellen tiefgezogenen Folie,
Figur 7 das Behälterunterteil mit Füllstandsdetektoren,
Figur 8 einen Schnitt durch das Behälterunterteil mit einem Verschluss in geschlossener Stellung,
Figur 9 einen Schnitt durch das Behälterunterteil mit dem Verschluss in der geöffneten Stellung,
Figur 10 eine schematische Darstellung einer Entleervorrichtung,
Figur 11 einen Schnitt durch ein anderes Ausführungsbeispiel des Behälterunterteils mit Verschlussmembran,
Figur 12 eine der Figur 11 entsprechende Darstellung bei geöffneter Verschlussmembran und
Figur 13 einen Schnitt durch ein weiteres Ausführungsbeispiel des Behälterunterteils mit einem anderen Behälterauslass

### Detaillierte Beschreibung

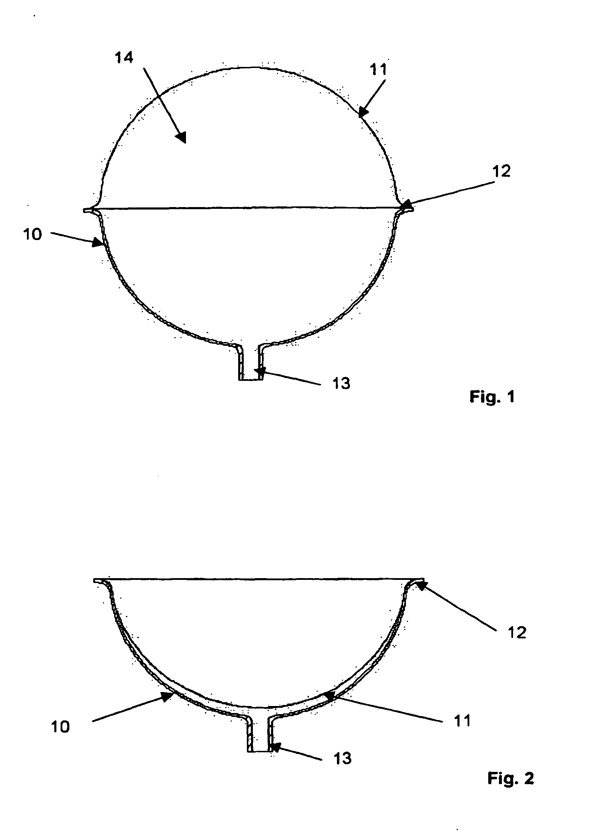

Figur 1 zeigt den Hauptteil eines Dosierbehälters **14** ohne Drucktank und ohne Verschluss. Das Unterteil **10** des Behälters **14** besteht aus einem massiven Kunststoffspritzteil und weist eine rotationssymmetrische (z.B. sphärische) Innenkontur auf. Am unteren Ende befindet sich ein hier nur schematisch gezeigter Auslass **13,** am oberen Ende ein umlaufender Flansch **12.**

Das Oberteil des Behälters **14** besteht aus einer dünnen Folie **11,** vorzugsweise mit einer Wandstärke zwischen 50 µm und 500 µm, die im Wesentlichen spiegelbildlich zur Innenkontur des Unterteils **10** geformt ist. Die Folie **11** besteht aus einem für die Füllsubstanz undurchlässigen Material, z.B. einer Kunststofffolie aus PE oder PET oder einer Kunststoff-Verbundfolie mit einer Aluminiumkaschierung. Die Formgebung der Folie **11** kann z.B. durch einen Tiefziehvorgang erfolgen.

Es gibt Materialien, die während der Lagerung einen bestimmten Anteil an Sauerstoff oder eines anderen Gases benötigen, damit sie nicht frühzeitig aushärten. Da dieses gelöste Gas im Laufe der Lagerzeit verbraucht wird, muss ständig aus der Umgebungsluft Sauerstoff nachgeliefert werden. Sofern die Folie **11** sauerstoffdurchlässig ist, erfolgt durch sie der Nachschub an Sauerstoff beständig, gleichmäßig und vor allem großflächig.

Ober- und Unterteil sind im Bereich ihrer größten Durchmesser an dem Flansch **12** durch Kleben oder Verschweißen luftdicht miteinander verbunden. Wird diese Vorrichtung blasenfrei durch ihren Ein- bzw. Auslass befüllt und luftdicht verschlossen, so erhält man einen hermetisch gegenüber der Umwelt abgeschlossenen Dosierbehälter. In diesen Behälter **14** kann nach Befüllung und Verschließen während der Lagerung oder des Transportes keine Luft oder Fremdsubstanz eindringen und kein Füllmaterial aus dem Behälter **14** entweichen. Auch durch extreme Temperaturwechsel bedingte Volumenveränderungen des Füllmaterials gleicht die dünne Folie **11** durch ihre Flexibilität vollständig aus, ohne dass in dem Behälter **14** selbst ein Über- oder Unterdruck entsteht.

Figur 2 zeigt den Behälter **14** vor dem Befüllen mit einer Flüssigkeit. Die flexible Folie **11** ist nach innen umgeklappt und liegt dicht an der Innenkontur des Unterteils **10** an. Ein Restvolumen an Luft im Auslass **13** wird durch Anlegen eines Vakuums abgesaugt. Anschließend wird unter Vakuum die Flüssigkeit in den Behälter **14** eingepresst.

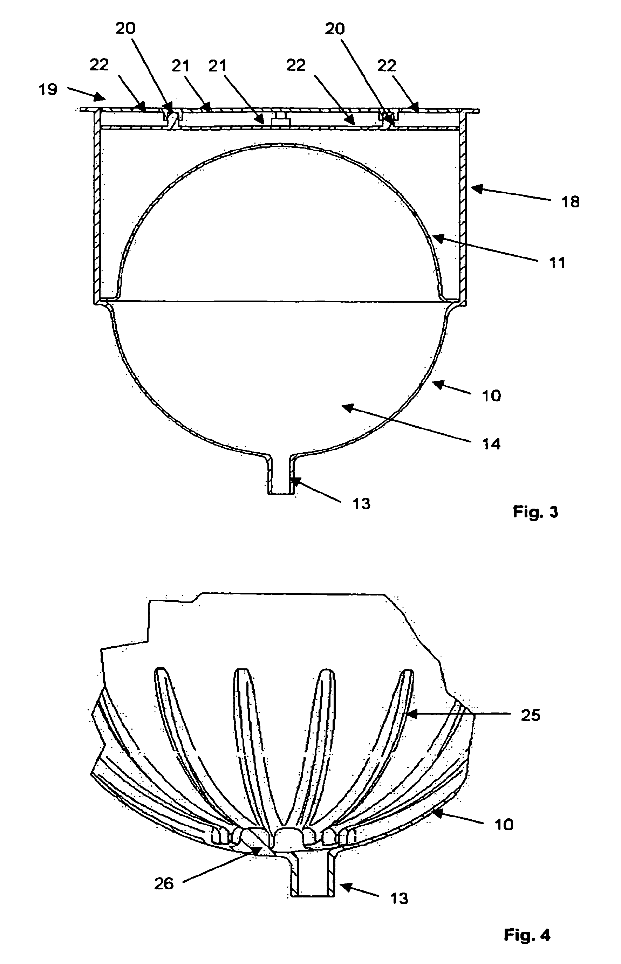

Figur 3 zeigt den Behälter **14** mit einem an den Flansch **12** angeformten oder dicht mit ihm verbundenen zylindrischen Gehäuse **18,** das an seinem oberen Ende durch einen doppelwandigen Deckel **19** abgeschlossen ist. Das Gehäuse **18** und der Deckel **19** dienen zum Schutz der flexible Folie **11** gegen mechanische Beschädigung und Lichteinfall. Der Deckel **19** besteht aus zwei durch mehrere Abstandsstücke **20** miteinander verbundenen Wandscheiben **21,** die nicht miteinander fluchtende Druckausgleichslöcher **22** aufweisen.

Das blasenfreie Befüllen des Behälters **14** erfolgt dadurch, dass die Folie **11** an die Innenkontur des starren Unterteils **10** gedrückt oder gesaugt wird. Das verbleibende Restvolumen wird durch den Auslass **13** einem Vakuum ausgesetzt, und anschließend wird der Behälter **14** von unten befüllt.

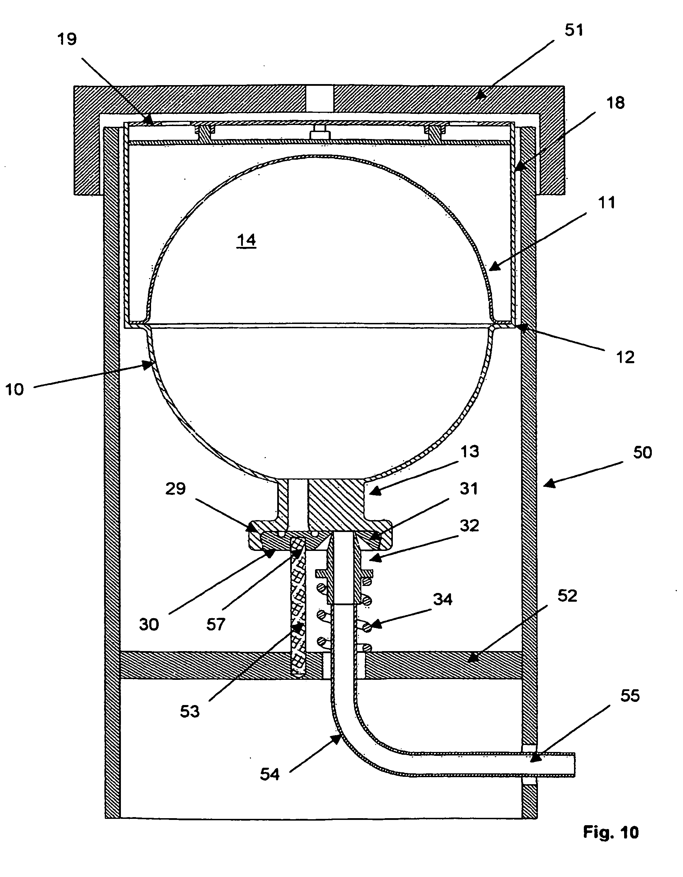

Zum Entleeren wird der Behälter **14** mit einer Druckkammer verbunden oder in eine solche eingebracht (wie weiter unten anhand von Figur 10 erläutert), die mindestens den obersten Bereich des Gehäuses **18** dicht umgibt. Die durch die Löcher **22** einströmende Druckluft lastet gleichmäßig auf der Folie **11** und presst die Flüssigkeit gleichmäßig über den Auslass **13** aus dem Behälter **14.** Sind das Gehäuse **18** und das Behälterunterteil **10** aus Gründen der Gewichts- und Kostenersparnis dünnwandig ausgebildet, so wird die Druckkammer zweckmäßig so gestaltet, dass sie zum Entleeren den gesamten Behälter **14** samt Gehäuse **18** mit Ausnahme des Auslasses **13** umschließt.

Die dosierte Entleerung des Behälters **14** erfolgt über eine gleichmäßige Druckbeaufschlagung der Folie **11** (z.B. mit Druckluft). Dabei ist das Eindringen von Luft in den Behälter **14** durch die dichte Randversiegelung am Flansch **12** ausgeschlossen. Die Folie **11** selbst wird sich während des gesamten Entleervorgangs sehr gleichmäßig verformen und keine Eigenkräfte aufbauen, da sie weder an einer Wandung reibt, noch durch eine Vorschubbewegung unkontrolliert gefaltet wird.

Erfolgt die Druckbeaufschlagung der Folie **11** mittels einer Hydraulikflüssigkeit, so eignet sich der Behälter **14** auch für eine volumetrische Dosierung.

Am Ende des Entleervorgangs wird die Folie **11** formschlüssig und faltenlos an der Innenkontur des Behälterunterteils **10** anliegen. Da sich die Folie **11** während des gesamten Entleervorgangs absolut kraftfrei verformt, werden auch die über eine feste Zeiteinheit ausgebrachten Dosiermengen bei einer konstanten Druckluft konstant bleiben.

Figur 4 zeigt das Unterteil **10** des Behälters **14** mit rinnenförmigen Vertiefungen **25,** die strahlenförmig auf den Auslass **13** des Behälters **14** zu verlaufen. Die Vertiefungen **25** stellen eine über der Zeit gleichmäßige Ausbringmenge der Flüssigkeit bis zum Zeitpunkt der vollständigen Entleerung sicher. Um auszuschließen, dass sich zum Ende des Entleervorgangs die Folie **11** vor die Öffnung des Auslasses **13** legt und die Restentleerung behindert oder verhindert, sind nahe dem Auslass **13** erhabene Stege **26** vorgesehen. Die Stege **26** halten die Folie **11** zur Öffnung des Auslasses **13** auf Abstand und sichern so das freie Abfließen der Flüssigkeit bis zur vollständigen Restentleerung des Behälters **14.**

Bei einer Fertigungsanlage die Klebstoff verarbeitet, benötigt man insbesondere zum Ende der Entleerung des Behälters eine Information über die noch verfügbare Restfüllmenge. Nur so lässt sich ein ordnungsgemäßer Behälterwechsel rechtzeitig durchführen, ohne dass es zu Fehldosierungen kommt. In herkömmlichen Kartuschen kann dies z.B. durch Detektieren der Stellung des Verschlusskolbens erfolgen. Bei dem hier beschriebenen Behälter **14** ist dies nicht ohne Weiteres möglich, da die Aufgabe der rückseitigen Abdichtung statt von einem starren Kolben von der flexiblen Folie **11** übernommen wird. Diese Folie wird sich während der Entleerung unregelmäßig verformen und kann somit nicht ohne Weiteres als Indikator für den Füllstand hergenommen werden. Gegen Ende der Entleervorgangs wird sich die Folie **11** jedoch an die Innenkontur des Unterteils anlegen.

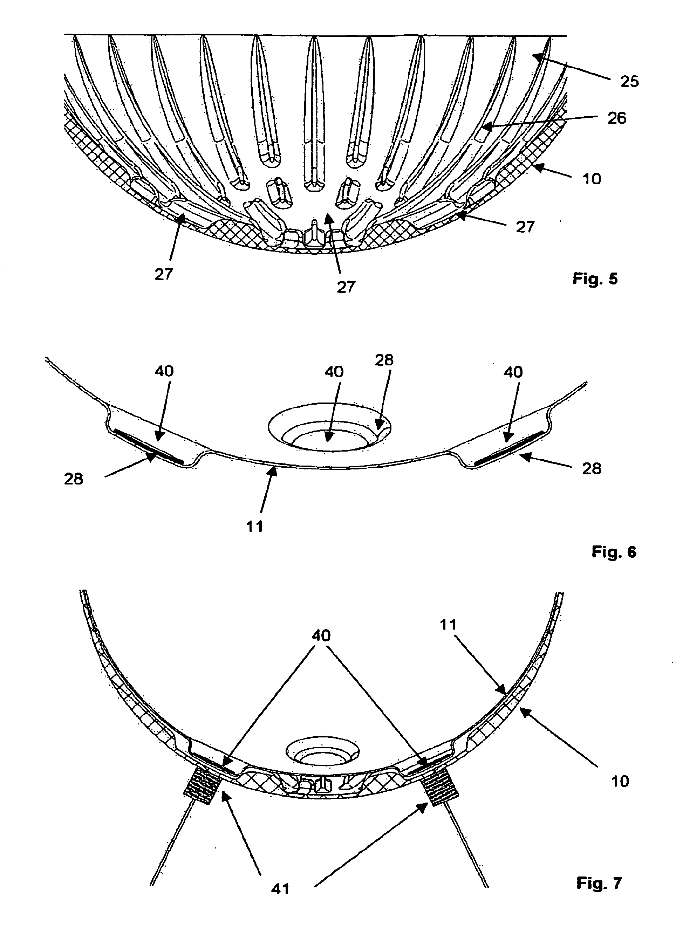

Bei dem Ausführungsbeispiel nach Figur 5 sind einige der Stege **26** in der Nähe des (hier nicht dargestellten) Auslasses **13** unterbrochen. In diesen Bereichen **27** sind an der Außenseite des Unterteils **10** induktiv oder kapazitiv arbeitende Sensoren **41** angebracht, deren Signale an einer (nicht gezeigten) Auswertschaltung liegen.

Wie in Figur 6 gezeigt, ist die Folie **11** an den Bereichen **27** des Behälterunterteils **10** mit Vertiefungen **28** versehen, in die beispielsweise kreisrunde Metallfolien **40** eingebracht sind.

Figur 7 zeigt das Unterteil **10** im entleerten Zustand, in dem die Folie **11** an der Innenwand des Unterteil **10** anliegt. In diesem Zustand werden die Metallfolien **40** von den Sensoren **41** detektiert.

Auf diese Weise wird das Anlegen der Folie **11** in der allernächsten Umgebung des Behälterauslasses **13** erfasst, so dass man einen Indikator für die Klebstoffrestmenge erhält. Da sich die Folie **11** jedoch nicht an allen Stellen gleichzeitig an die Behälterwand anlegen wird, wird die Stellung der Folie **11** an mehreren (z.B. vier) Positionen am Umfang abgefragt. Die Auswertschaltung kann so arbeiten, dass sie eine Vorwarnung zum Behälterwechsel abgibt, wenn sich die Folie **11** an einer dieser Positionen anlegt. Liegt sie zum Beispiel an drei Positionen an, so kann das System die gesamte Anlage abschalten, um Fehldosierungen zu vermeiden.

Soll die Blasenfreiheit der Füllsubstanz über den gesamten Dosierprozess bis zum Austreten der Substanz aus einem nachgeschalteten Dosierventil erhalten bleiben, muss sichergestellt sein, dass beim Ankoppeln des Behälters **14** an den Produktschlauch einer Dosieranlage keine Luft eingebracht wird.

Zudem ergibt sich durch die Folie **11** beim Einsatz marktüblicher Kartuschenverschlüsse ein zusätzliches Problem beim Öffnen des beschriebenen Behälters **14.** So wird bei niedrigviskosen Produkten beim Entfernen einer Verschlusskappe bei nach unten gehaltenem Auslass das Füllmaterial auslaufen, da die flexible Folie **11** keinen Halt wie ein Kartuschenkolben bietet. Wird der Auslass noch oben gehalten, saugt dagegen der Behälter **14** wegen des Gewichtes der Füllsubstanz und der flexiblen Folie **11** Luft an.

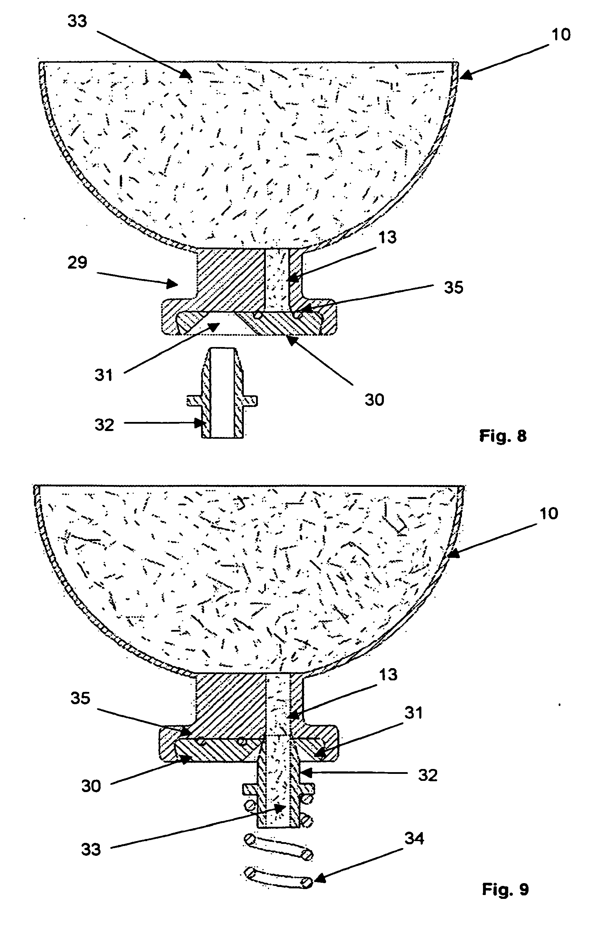

Zur Lösung dieses Problems ist, wie in Figur 8 gezeigt, am Ende des Auslasses **13** am Unterteil **10** des Behälters **14** eine Scheibe **30** vorgesehen, die mit ihrer planen Fläche den Auslass **13** luftdicht verschließt. An dieser Fläche steht blasenfrei die Flüssigkeit **33** des Behälters **14** an. Größtmögliche Dichtigkeit während der Lagerung und des Transportes bietet eine den Auslass **13** umgebende Dichtungslippe **35** (z.B. ein O-Ring).

Die rotationssymmetrische Scheibe **30** ist um eine bezüglich des Auslasses **13** versetzte Achse drehbar gelagert. Die Scheibe **30** weist eine Durchgangsöffnung **31** auf, in die ein Nippel **32** eines Produktschlauchs eingeführt werden kann. Die Durchgangsöffnung **31** verjüngt sich von außen nach innen (Figur 8 und 9 von unten nach oben) konisch oder kalottenförmig in der Form, dass die Differenz zwischen dem Durchmesser der Durchgangsöffnung **31** und dem des Nippels **32** von einem zunächst positiven Wert ausgehend mindestens Null oder kleiner wird. Diese spezielle Form der Durchgangsöffnung **31** verhindert die Bildung eines Luftpolsters beim Einführen des Nippels **32.** Der Produktschlauch führt zu dem eigentlichen, nachgeschalteten (nicht gezeigten) Dosierventil.

Durch eine Drehbewegung der Scheibe **30** um ihre Achse wird der Nippel **32** direkt unter den Auslass **13** des Behälters **14** gebracht und durch eine Feder **34** ein kleines Stück in die Öffnung **31** hineingepresst (Figur 9). Sofern der Produktschlauch vollständig mit Flüssigkeit befüllt war, ist sichergestellt, dass keine Luft beim Anschließen der Produktleitung eingebracht wird. Da so der Auslass **13** zu keinem Zeitpunkt gegenüber der Außenwelt geöffnet ist, kann niemals Material ausfließen oder Luft angesaugt werden. Der oben beschriebene Folieneffekt kann sich somit nicht auswirken. Das Anschließen und auch das Austauschen eines Dosierbehälters **14** erfolgen blasenfrei.

In der Praxis können Dosierbehälter **14** während eines Produktionstages auch nur teilweise entleert werden. Diese teilgefüllten Behälter müssen jedoch über Nacht, über das Wochenende oder bis zum nächsten Produktionsauftrag gekühlt oder tiefgefroren eingelagert werden. Auch beim Entfernen und Wiederanschließen von teilgefüllten Dosierbehältern **14** verhindert die in Figur 8 und 9 gezeigte Anordnung das Eindringen ungewollter Luftblasen in die Produktleitung und sorgt für einen verlässlichen, störungsfreien Produktionsprozess.

Die in Figur 10 schematisch dargestellte Entleervorrichtung umfasst ein Druckgefäß **50** mit einem bajonettartig verschließbaren Deckel **51,** wie es etwa aus EP 0 532 945 A1 bekannt ist. Der Nippel **32** stützt sich über die Druckfeder **34** an einer Zwischenwand **52** ab, die auch einen nach oben ragenden Arretierstift **53** trägt. Ein mit dem Nippel **32** verbundener Produktschlauch 54 führt zu einem äußeren Anschlussstutzen **55.**

Das Druckgefäß **50** hat einen solchen Innendurchmesser, dass es den eingesetzten Behälter **14** mit wenig Spiel umgibt. Der Behälter **14,** der mit dem in Figur 3 gezeigten Gehäuse **18** versehen ist, wird beim Schießen des Deckels **51** gegen den von der Feder **34** vorgespannten Nippel **32** nach unten gedrückt, so dass sich dieser in der konischen oder kalottenförmigen Durchgangsöffnung **31** zentriert. Gleichzeitig greift der Arretierstift **53** in ein in der Verschlussscheibe **30** des Behälters **14** vorhandenes Sackloch **57** ein. Zur anschließenden Verriegelung wird der Deckel **51** gegenüber dem Druckgefäß **50** gedreht, wobei er den Behälter **14** mitnimmt, während die Scheibe **30** durch den Arretierstift **53** festgehalten wird. Auf diese Weise wird gleichzeitig mit dem Schließen des Druckgefäßes **50** der Behälter **14** geöffnet und kann nun durch Einleiten von Druck in das Gefäß 50 über den Produktschlauch **54** entleert werden.

Der nach oben offene Nippel **32** verhindert ein Auslaufen der Flüssigkeit aus dem Produktschlauch **54,** was bei losen oder nach unten offenen Produktleitungen passieren würde. Des Weiteren gestattet diese Anordnung den Verzicht auf ein Ventil im Nippel **32,** welches den Volumenstrom behindern und zu einem höheren Reinigungsaufwand führen würde.

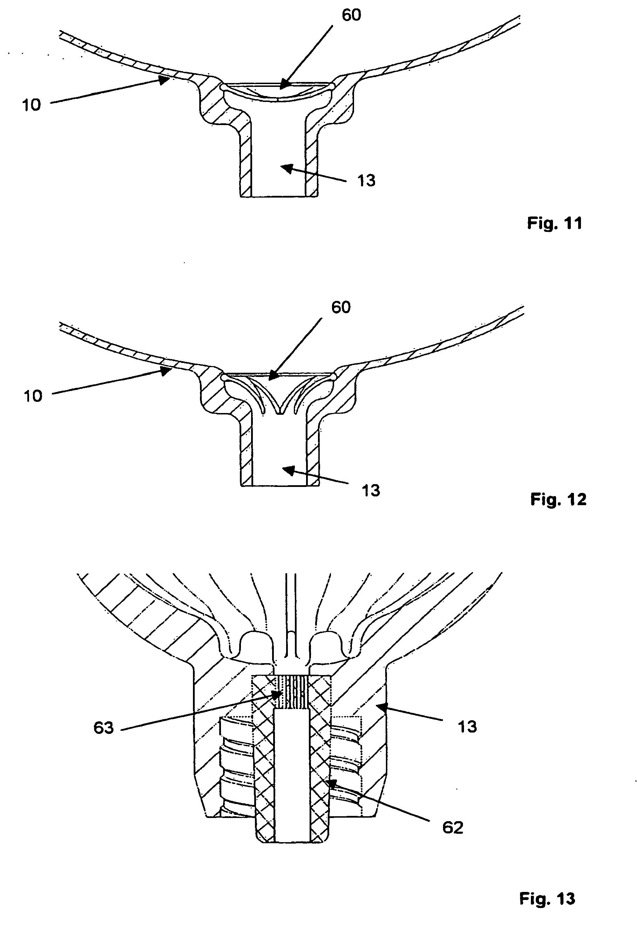

Bei einem alternativen Ausführungsbeispiel ist direkt am Auslass **13** des Behälters **14** eine geschlitzte, weiche, elastische Verschlussmembran **60** befestigt. Figur 11 zeigt das Unterteil **10** mit der Membran **60** in geschlossener, Figur 12 in geöffneter Stellung. Die Membran **60** bietet ausreichenden Widerstand gegen unbeabsichtigtes Auslaufen der Flüssigkeit oder Ansaugen von Luft während der Montage.

In einer weiteren, in Figur 13 gezeigten Alternative ist anstelle der Membran **60** in den Auslass **13** ein Einsatzkörper **62** mit feinen Durchgangsbohrungen (Kapillaren) 63 (z.B. 0,1 bis 1,0 mm Durchmesser) eingefügt, die auf Grund ihres Durchflusswiderstandes ein unbeabsichtigtes Auslaufen der Flüssigkeit verhindern.

### Bezugszeichenliste

- 10: Unterteil
- 11: Folie
- 12: Flansch
- 13: Auslass
- 14: Dosierbehälter
- 18: Gehäuse
- 19: Deckel
- 20: Abstandsstücke
- 21: Wandscheiben
- 22: Druckausgleichslöcher
- 25: rinnenförmige Vertiefungen
- 26: Stege
- 27: unterbrochene Bereiche
- 28: Vertiefungen
- 29: Verschluss
- 30: Scheibe
- 31: Durchgangsöffnung
- 32: Nippel
- 33: Flüssigkeit
- 34: Feder
- 35: Dichtungslippe
- 40: Metallfolien
- 41: Sensoren
- 50: Druckgefäß
- 51: Deckel
- 52: Zwischenwand
- 53: Arretierstift
- 54: Produktschlauch
- 55: Anschlussstutzen
- 57: Sackloch
- 60: Verschlussmembran
- 62: Einsatzkörper
- 63: Durchgangsbohrung

## Patentansprüche

1. Behälter für fließfähige Substanzen mit einem starren konkaven im Wesentlichen halbkugelförmigen Unterteil (**10**), das mit einem Auslass (**13**) versehen ist, einem mit dem Unterteil (**10**) im Bereich des größten Durchmessers luftdicht verbundenen Oberteil (**11**), das aus einer zur Innenkontur des Unterteils (**10**) im Wesentlichen spiegelbildlich konvex geformten flexiblen Folie besteht, und einem das Oberteil (**11**) umschließenden Gehäuse (**18**), das mit einem Flansch des Unterteils (**10**) dicht verbunden und zur Druckentleerung des Behälters für den Eintritt von Druckluft oder Hydraulikflüssigkeit geöffnet ist.

2. Behälter nach Anspruch 1, wobei das Gehäuse (**18**) durch einen doppelwandigen Deckel (**19**) abgeschlossen ist, dessen beide Wandscheiben (**21**) nicht miteinander fluchtende Druckausgleichslöcher (**22**) aufweisen.

3. Behälter nach Anspruch 1 oder 2, wobei das Gehäuse (**18**) an den Flansch (**12**) angeformt ist.

4. Behälter nach Anspruch 3, wobei das Gehäuse (**18**) zylindrisch ist.

5. Behälter nach einem der vorhergehenden Ansprüche, wobei die das Oberteil (**11**) bildende Folie sauerstoffdurchlässig ist.

6. Behälter nach einem der vorhergehenden Ansprüche, wobei das Unterteil (**10**) innen rinnenförmige Vertiefungen (**25**) aufweist, die strahlenförmig auf den Auslass (**13**) zu verlaufen.

7. Behälter nach Anspruch 6, wobei die Vertiefungen (**25**) eine in Richtung des Auslasses (**13**) zunehmende Tiefe haben.

8. Behälter nach einem der vorhergehenden Ansprüche, wobei das Unterteil (**10**) mindestens einen nahe dem Auslass (**13**) angeordneten, nach innen ragenden Vorsprung (**26**) aufweist.

9. Behälter nach einem der vorhergehenden Ansprüche, wobei am Unterteil (**10**) ein Sensor (**41**) angeordnet ist, der das Anliegen der Folie (**11**) an der Innenwand des Unterteils (**10**) erfasst.

10. Behälter nach Anspruch 9, wobei der Sensor (**41**) mit einem auf der Folie (**11**) angebrachten metallischen Körper (**40**) induktiv oder kapazitiv koppelbar ist.

11. Behälter nach Anspruch 10, wobei mehrere Sensoren (**41**) nahe dem Auslass (**13**) positioniert sind

12. Behälter nach einem der vorhergehenden Ansprüche, wobei der Auslass (**13**) einen Verschluß (**29**) mit einer Scheibe (**30**) aufweist, die um eine zur Öffnung des Auslasses (**13**) versetzte Achse drehbar ist und eine Durchgangsöffnung (**31**) zum Einführen eines Entnahmenippels (**32**) aufweist.

13. Behälter nach einem der Ansprüche 1 bis 11, wobei der Auslass (**13**) durch eine geschlitzte Membran (**60**) verschlossen ist.

14. Behälter nach einem der Ansprüche 1 bis 11, wobei der Auslass (**13**) mit mindestens einer Durchgangskapillare (**63**) versehen ist.

15. Verfahren zum blasenfreien Dosieren fließfähiger Substanzen mittels eines Behälters (**14**) nach einem der vorhergehenden Ansprüche, **dadurch gekennzeichnet, dass** das Gehäuse (**18**) an eine Druckkammer angeschlossen wird.

## Claims

1. A container for flowable substances, comprising a rigid, concave, substantially hemispherical lower part (**10**) provided with an outlet (**13**), an upper part (**11**) connected to the lower part (**10**) in an air-tight manner in the region of the largest diameter and consisting of a flexible film having a convex shape substantially mirror-inverted with respect to the inner contour of the lower part (**10**), and a body (**18**) enclosing the upper part (**10**), connected to a flange of the lower part (**10**) and open for the entry of pressurised air hydraulic liquid for emptying the container by pressure.

2. The container of claim 1, wherein the body (**18**) is closed by a double-walled cover (19) the two disc-shaped walls (**21**) of which have non-aligned pressure equalising holes (**22**).

3. The container of claim 1 or 2, wherein the body (**18**) is formed integrally with the flange (**12**).

4. The container of claim 3, wherein the body (**18**) is cylindrical.

5. The container of any preceding claim, wherein the film forming the upper part (**11**) is permeable to oxygen.

6. The container of any preceding claim, wherein the lower part (**10**) has inner grooves (**25**) extending radially to the outlet (**13**).

7. The container of claim 6, wherein the grooves (**25**) have depths decreasing toward the outlet (**13**).

8. The container of any preceding claim, wherein the lower part (**10**) has at least one inward protruding projection (**26**) disposed near the outlet (**13**).

9. The container of any preceding claim, comprising a sensor (**41**) provided at the lower part (**10**) for detecting an abutment of the film (**11**) at the inner wall of the lower part (**10**).

10. The container of claim 9, wherein the sensor (**41**) is adapted for inductive or capacitive coupling with a metallic body (**40**) provided on the film (**11**).

11. The container of claim 10, comprising a plurality of sensors (**41**) positioned near the outlet (**13**).

12. The container of any preceding claim, wherein the outlet (**13**) has a closure (**29**) with a disk (**30**) which is rotatable about an axis offset from the opening of the outlet (**13**) and has a passage for receiving a dispensing nipple (**32**).

13. The container of any one of claims 1 to 11, wherein the outlet (**13**) is closed by a slotted membrane (**60**).

14. The container of any one of claims 1 to 11, wherein the outlet (**13**) is provided with at least one lead-through capillary (**63**).

15. A method for bubble-free metering flowable substances by means of a container (**14**) according to any preceding claim, **characterised in that** the body (**18**) is connected to a pressure chamber.

## Revendications

1. Récipient pour substances susceptibles de s'écouler, comprenant une partie inférieure (10) rigide, concave et sensiblement en forme de demi-sphère, qui est pourvue d'une sortie (13), d'une partie supérieure (11) reliée, de manière étanche à l'air, à la partie inférieure (10) dans la zone du plus grand diamètre, et qui est constituée d'une feuille flexible formée selon une configuration convexe sensiblement symétrique au contour intérieur de la partie inférieure (10), et l'ensemble comprenant également un carter (18) qui entoure la partie supérieure (11), et est relié de manière étanche à une bride de la partie inférieure (10) tout en étant ouvert pour permettre l'entrée d'air comprimé ou d'un fluide hydraulique en vue de la vidange sous pression du récipient.

2. Récipient selon la revendication 1, dans lequel le carter (18) est fermé par un couvercle (19) à paroi double, dont les deux disques de paroi (21) présentent des trous de compensation de pression (22), qui ne sont pas mutuellement alignés.

3. Récipient selon la revendication 1 ou la revendication 2, dans lequel le carter (18) est formé par moulage sur la bride (12).

4. Récipient selon la revendication 3, dans lequel le carter (18) est cylindrique.

5. Récipient selon l'une des revendications précédentes, dans lequel la feuille formant la partie supérieure (11) est perméable à l'oxygène.

6. Récipient selon l'une des revendications précédentes, dans lequel la partie inférieure (10) présente, à l'intérieur, des creux (25) en forme de goulotte, qui s'étendent en forme de rayons en direction de la sortie (13).

7. Récipient selon la revendication 6, dans lequel les creux (25) présentent une profondeur croissante en direction de la sortie (13).

8. Récipient selon l'une des revendications précédentes, dans lequel la partie inférieure (10) présente au moins une protubérance (26) agencée à proximité de la sortie (13) et faisant saillie vers l'intérieur.

9. Récipient selon l'une des revendications précédentes, dans lequel sur la partie inférieure (10) est agencé un détecteur (41) qui relève l'application de la feuille (11) contre la paroi intérieure de la partie inférieure (10).

10. Récipient selon la revendication 9, dans lequel le détecteur (41) peut être couplé par voie inductive ou capacitive avec un corps métallique (40) placé sur la feuille (11).

11. Récipient selon la revendication 10, dans lequel plusieurs détecteurs (41) sont positionnés à proximité de la sortie (13).

12. Récipient selon l'une des revendications précédentes, dans lequel la sortie (13) est pourvue d'un système de fermeture (29) comprenant un disque (30), qui peut tourner autour d'un axe décalé par rapport à l'ouverture de la sortie (13) et présente une ouverture de passage (31) pour l'insertion d'un embout de prélèvement (32).

13. Récipient selon l'une des revendications 1 à 11, dans lequel la sortie (13) est fermée par une membrane (60) fendue.

14. Récipient selon l'une des revendications 1 à 11, dans lequel la sortie (13) est pourvue d'au moins un capillaire de passage (63).

15. Procédé pour doser sans bulles des substances susceptibles de s'écouler, au moyen d'un récipient (14) selon l'une des revendications précédentes, **caractérisé en ce que** l'on raccorde le carter (18) à une chambre de pression.
